# EUROPEAN PATENT SPECIFICATION

(11) **EP 3 852 522 B1**
(45) Date of publication and mention of the grant of the patent: **06.11.2024**
(21) Application number: 19863075.8
(22) Date of filing: 22.09.2019
(51) Int. Cl.: A01M 1/02, A01K 67/033, G06V 20/69

(54) **MOSQUITO CLASSIFICATION, SORTING, AND STERILIZATION**
KLASSIFIZIERUNG, SORTIERUNG UND STERILISATION VON MÜCKEN
CLASSIFICATION, TRI ET STÉRILISATION DE MOUSTIQUES

(30) Priority: 21.09.2018 US 201862734300 P; 07.01.2019 US 201962789385 P
(43) Date of publication of application: 28.07.2021
(73) Proprietor: Senecio Ltd., 4463107 Kfar-Saba (IL)
(72) Inventor: LEPEK, Hanan, 4463107 Kfar-Saba (IL); NAVE, Tamir, 5545806 Kiryat-Ono (IL); MATARASSO, Hasdi, 3708034 Pardes Hana (IL)
(74) Representative: ip21 Ltd
(86) International application number: PCT/IL2019/051047
(87) International publication number: WO 2020/058982

(56) References cited:
- WO-A1-2018/134829
- No further relevant documents disclosed
- B J SMITTLE , R S PATTERSON : "Container for irradiation and mass transport of adult mosquitoes", MOSQUITO NEWS , vol. 34, no. 4, 31 December 1974 (1974-12-31), pages 406 - 408, XP055796012

## Description

### FIELD AND BACKGROUND OF THE INVENTION

The present invention, in some embodiments thereof, relates to an apparatus, system and method for insect counting, classification, sorting and/or sterilization and, more particularly, but not exclusively, to a such a system for mosquitoes.

The Sterile mosquitoes technique (SIT), in which sterile male mosquitos are released for mating has proved to be an efficient tool for suppression of local mosquito populations.

Sterility of the males may be achieved *inter alia* by placing the mosquitoes in the presence of radiation for a preset amount of time, not too short so it has no effect and not too long so as to harm or even kill them.

The problem is that while it is fairly easy to handle mosquitoes at the egg, larva or pupa stage, the adults may fly, and are not going to remain still for the preset amount of time for them to receive the inducing X-Rays or gamma rays at the required dose and or at the required distance from the radiation source in order to render the mosquitoes sterile, without having negative effects on their competitiveness and even Longevity.

It is possible to irradiate the insects at the pupa stage or larva stage. However, then different problems arise, namely that special X-Ray cabinets and mountings are required in order to handle pupa trays, in order to distribute the radiation homogenously. Furthermore, due to low penetration in water, irradiating pupa would require very shallow water or no water at all with pupae being placed in moisture, which may also have negative effects on the development of the mosquito into a competitive adult mosquito, being able to compete in nature against wild type male mosquitoes.

In addition, X-Ray radiation, and for that matter, gamma radiation from say a cobalt 60 source, are not necessarily distributed uniformly over a designated area, or over short time spans, so that having a tray or a canister holding pupae or larva inside, may create a situation in which different mosquitoes at different locations within that tray or canister, received different dosages, resulting in a possible situation in which not all of the mosquitoes have been sterilized. In order to reach 100% sterility, the radiation power or exposure time may be increased, but due to the lack of uniformity, now some mosquitoes may receive an excessive dosage and their competitiveness may be harmfully affected.

When using a radiation chamber, studies show that it is important to confine the insects in a small volume in the center of the irradiation chamber to ensure a dose uniformity ratio of < 1.1 (where dose uniformity = highest dose/lowest dose) ("Radiation biology of mosquitoes", Malaria Journal, Michelle EH Helinski, Andrew G Parker and Bart GJ Knols, dated 16 November 2009).

Different studies suggest, that for example, in the case of *aedes albopictus,* when trying to sterilize the pupae by utilizing X-Rays, having a dose of up to 40gy will result in female sterilization lower than 100%. At 40gy, the sterility observed is 99%. However, when the sterility for females is 100% at above the 40gy dose level, then negative effects on the longevity of the adult males are observed, suggesting also lower competitiveness ("X-Ray-Induced Sterility in Aedes albopictus and Male Longevity Following Irradiation", H. YAMADA, A. G. PARKER, C. F. OLIVA, F. BALESTRINO, AND J.R.L. GILLES, Entomological Society of America, 2014). The same effect hardly happens when sterilization is carried out on adults.

For the *Anopheles gambiae* mosquito species, as indicated by "Radiation biology of mosquitoes", Malaria Journal, Michelle EH Helinski, Andrew G Parker and Bart GJ Knols, dated 16 November 2009, when pupae (22-27 hours old) and adults (< 24 hours old) of *Anopheles gambiae* were irradiated with a high dose of 120 Gy, an increased mortality for the irradiated pupae 24h after irradiation was reported compared to zero mortality in the irradiated adults. This again emphasizes the benefits of sterilizing mosquitoes at the adult stage, but the fact is that to date this remains a major challenge as indicated by "Radiation biology of mosquitoes", Malaria Journal, Michelle EH Helinski, Andrew G Parker and Bart GJ Knols, 16 November 2009: "Adults, however, are much more fragile and require careful handling. Prior to irradiation, adults can be inactivated by chilling which allows them to be confined in a small space within the irradiator so that dose variation can be reduced and mechanical damage to the insects minimized. In small scale studies, adults can usually tolerate the chilling and stacking for the irradiation but in operational campaigns very large numbers of insects will have to be irradiated and new protocols will be required."

Hence there is a real problem that on the one hand, ease of handling makes it a preferred way today to irradiate mosquitoes at the pupa stage, but on the other hand this effects their longevity and competitiveness. It is best to irradiate mosquitoes once they completely finished their development, preferably at the adult stage, but for mass production in commercial SIT operations, in which millions of mosquitoes are required to be sterilized and released on a daily basis, then the difficulty of handling the fragile mosquitoes make the current methods for sterilizing adult mosquitoes impractical, hence the SIT method is not currently used at large scale.

It is noted that such negative effects on longevity is similar whether the radiation dose is delivered using X Rays or gamma rays.
To summarize there are three major problems:
1. It is preferred to have the same dosage level delivered to all mosquitoes being irradiated, and if dose delivery is not uniform, e.g. due to different distance of the insect from the radiation source, or inherent limitations of the radiation distribution pattern and the system design etc. then in order to verify all are sterile, some mosquitoes will experience a higher dose than required.
2. At a high dose rate, the exact dose rate being species dependence, for example above 40gy for *aedes albopictus,* mosquito competitiveness is harmfully affected.
3. For commercial large scale operations, a time efficient and continuous or quasi-continuous process that can sterilize large numbers of adults would be desirable.

The Mosquito Sterile Insect technique typically requires the release of exclusively male mosquitoes. For this reason, mosquito labs classify mosquitoes by their gender and then sort them (extract the females) prior for release in the field.

To date, a popular method for mosquito sex sorting is the sorting of the mosquito during the pupal stage using a sieving device, based on the size differences between male and female pupae.

However, relying on such a statistical method may result in female contamination in the release boxes. Such contamination is undesirable as the females act as disease vectors, and local regulations may limit the amount of female contamination that is allowed before the system can be used.

WO2018 134829A1 discloses a method and apparatus for mechanical sex-sorting of mosquitoes by extracting a class of mosquitoes from unsorted mosquitoes comprising obtaining unsorted mosquitoes, obtaining images of individual mosquitoes in a stationary phase, electronically classifying the individuals from the images into male mosquitoes and/or female mosquitoes, and possibly also unclassified objects; obtaining co-ordinates of individuals of at least one of the male mosquito and female mosquito classifications, and using a robot arm to reach an individual identified by the obtained coordinates to store or remove the individuals, thereby to provide sex-sorted mosquitoes.

### SUMMARY OF THE INVENTION

The present embodiments may provide an apparatus, system and a method that applies operations such as counting, classification, sorting and sterilizing to insects which are substantially immobilized and rendered distinct, thus allowing the operations to be efficient and measured. The present embodiments may enable switching between immobilized and mobilized insect status substantially rapidly, as opposed to cooling systems, in which if mosquitoes are immobilized at 6-8 degree Celsius, then after shutting the cooling, or moving the mosquitoes to a non -cooled area, it may take as much as half a minute or more for the mosquitoes to start walking and then flying. A semi-automated counting, classification or and sorting process, carried out on the individuated insects may be based on combining the advantages of personal and automatic sorting, and irradiation operations may rely on individuation of the insects in order to apply measured doses.

According to an aspect of some embodiments of the present invention there is provided a method of immobilizing and applying an operation to insects, comprising:
obtaining insects;
directing said insects to a first side of a permeable surface;
applying a pressure gradient through said permeable surface to be lower at a second side of said surface than at said first side, thereby to immobilize said insects on said permeable surface said pressure gradient thereby being effected as a pull of said insects to said permeable surface to immobilize said insects on said permeable surface;
conveying said insects on said permeable surface under continuing application of said pressure gradient; and
carrying out said operation on said immobilized insects.
In an embodiment, said applying a pressure gradient comprises applying suction across said permeable surface from said second side, or applying overpressure from said first side.

In an embodiment, said applying a pressure gradient comprises applying a first relatively low pressure gradient to allow insects to walk and separate but be unable to fly, and second relatively high pressure gradient that prevents said insects from walking.

In an embodiment, said permeable surface is at least part of a moving conveyor having a conveying length along which said insects are conveyed, the pressure gradient being applied along said conveying length.

Embodiments may comprise applying respectively different pressure gradients at different locations along said conveying length, and/or applying an intermediate pressure gradient being higher than said first pressure gradient and lower than said second pressure gradient.

In an embodiment, said pressure gradient is reversible.

In an embodiment, said operation comprises counting insects on said permeable surface and/or applying a larvicide.

In an embodiment, said operation comprises obtaining an image or a position of an individual insect.

In an embodiment, said operation comprises applying a predetermined radiation dosage to an individual insect.

The method may involve focusing a beam from a radiation source onto said individual insect according to an obtained position.

In an embodiment, said operation comprises determining a sex of an individual insect.

In an embodiment, said operation comprises removing an individual insect if determined to be of a first sex and/or irradiating said individual insect if determined to be of a second sex.

In an embodiment, said operation comprises applying an unfocused radiation dose substantially evenly over said permeable surface to reach each of said separated insects.

In an embodiment, said obtaining said mass of insects comprises causing said insects to fly through a duct with an airflow to a collection area.

In an embodiment, said obtaining said mass of insects comprises collecting said mass of insects when immobilized by cooling or using an anaesthetizing agent.

Embodiments may involve warming said insects to remobilize said insects and allow separation, prior to said increasing said pressure.

Embodiments may involve applying said operation at an operation station, said insects being conveyed up to said operation station and then kept stationary for said operation.

Embodiments may involve applying said operation at an operation station while said insects are conveyed past said operation station.

In an embodiment, said operation comprises sterilization, in particular sweeping a sterilization beam over said insects.

In an embodiment, the operation comprises classification, and particularly using an algorithm to classify an insect according to sex, applying a score to said classification and if said score is below a predetermined threshold then referring said insect to a human operator.

In an embodiment, the operation comprises classification, and particularly using an algorithm to classify an insect according to sex, and using a graphical user interface to display respective classifications to a human operator for verification.

In an embodiment, said operation comprises removing an individual insect if determined to be of a first sex and/or irradiating said individual insect if determined to be of a second sex and a decision regarding said removing or irradiating is obtained via said graphical user interface.

In an embodiment, the operation comprises sterilization, said sterilization comprising moving at least one radiation source in two dimensions in a plane above said permeable surface.

In an embodiment, the operation comprises sterilization, said sterilization comprising moving at least two radiation sources to provide a focused beam or a homogenous beam at said permeable surface.

In an embodiment, the operation comprises sterilization and said predetermined dose is provided in a plurality of fractional doses with intervals therebetween.

The apparatus may include one or more of: a camera for obtaining images and/or coordinates of said insects, one or more robot actuated devices for removing identified ones of said insects based on provided coordinates, one or more radiation sources configured to provide a predetermined radiation dose to a specified location, and a graphical user interface, the graphical user interface providing interactions to allow an operator to control said operations.

The graphical user interface may comprise:
a first view showing an area of said permeable surface with insects labeled according to a classification or as unclassified and allowing for selection between said insects; and
a second view showing an enlargement of a selected insect in said first view.

The graphical user interface may have a classification or counting interaction to input a classification of a currently selected insect.

In an embodiment, said graphical user interface comprises at least one operation interaction to apply an operation to a currently selected insect.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention, in particular relating to identifying and then directing controlled implements to sort the insects, can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE SEVERAL VIEWS OF THE DRA WING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 is a simplified diagram illustrating a mass of insects on a porous or permeable conveyed surface according to the present embodiments and showing the mass before and after disentangling;
Fig. 2 is a simplified diagram showing a vacuum source under a porous conveyed surface according to the present embodiments;
Fig. 3 is a simplified schematic block diagram illustrating a sterilization operation using the present embodiments;
Fig. 4 is a simplified diagram illustrating a conveyor according to the present embodiments with a porous or permeable conveyed surface;
Fig. 5 is a simplified diagram showing a pressure source and sterilization apparatus placed in relation to the conveyor of Fig. 4;
Fig. 6 is a simplified diagram illustrating sweep of a sterilization beam over the conveyed permeable surface of the present embodiments;
Fig. 7 is a simplified schematic diagram illustrating apparatus according to the present embodiments in which a pupa tray provides insects via a guide tunnel for the sterilization operation according to the present embodiments;
Fig. 8 is a simplified diagram illustrating the use of cold air as a first immobilization source for the insects being delivered and a pressure gradient as a second immobilization source for the conveyor, according to embodiments of the present invention;
Fig. 9 is a simplified diagram illustrating insects being conveyed directly to the operation area in accordance with embodiments of the present invention;
Fig. 10 is simplified diagram showing a layout for a conveyor having a sterilization point and a loading point, according to embodiments of the present invention;
Fig. 11 is a simplified diagram illustrating use of a sweeping sterilization beam on insects on a conveyor that moves perpendicularly to the sweep direction according to embodiments of the present invention;
Fig. 12 is a simplified diagram showing insects being shunted aside from a moving conveyor for an operation to be carried out according to embodiments of the present invention;
Fig. 13 is a simplified diagram showing beams being focused on particular insects according to embodiments of the present invention;
Fig. 14 is a simplified diagram illustrating regions of high and low dose radiation according to embodiments of the present invention;
Fig. 15 is a view along the conveyor of the beam focusing embodiment of Fig. 13;
Figs. 16A - 16C are an overall view and two details of a work station in which an operator manages insects according to embodiments of the present invention;
Figs. 17 -19 are three views of a screen of a GUI according to embodiments of the present invention to allow an operator to interact with the various operations carried out in the insects; and
Fig. 20 is a simplified diagram illustrating a conveyor where a pressure gradient is controlled at multiple points along the conveyor, according to embodiments of the present invention.

### DESCRIPTION OF SPECIFIC EMBODIMENTS OF THE INVENTION

The present invention, in some embodiments thereof, relates to an apparatus, system and method for insect counting, classification, sorting and/or sterilization and, more particularly, but not exclusively, to a such a system for mosquitoes.

A method of applying an operation to an individually separated and immobile insect comprises obtaining a mass of insects, or at least two insects, directing the mass of insects onto a permeable surface, and applying a pressure gradient through the permeable surface to apply suction of the insects down into the surface, to allow the insects to become substantially immobilized within seconds down to even a friction of a second when using on/off vacuum table. The operation, whether it be counting, classification, sorting or sterilization, is then carried out on the immobilized insects. A corresponding apparatus and a graphical user interface for controlling the operation, is also provided.

It is noted that the sizing of a mesh forming a permeable surface, specifically for mosquitoes. may be smaller than a few mm per spacing, otherwise the insects would be sucked through them.

Two levels of pressure gradient may be applied, a first level that allows them to walk but not to fly and a second level that makes them immobile. The two different levels may be different for different species of insect and for each application. Also the immobilization pressure may be switched within a fraction of a second if needed, between the two states or between on and completely off states. If the insects are tangled together then the first level can be used to allow the insects to separate from each other.

A conveyor may refer to many options for conveying materials and particularly insects, including circular movement, and linear movement. Some embodiments may use rails. Different kinds of permeable surface may enable suction to be experienced at the upper side of the surface. The porous surface may be a net, or a mesh like surface for example. For the porous surface, the spacings may be small enough to prevent the insect being sucked through them towards the suction unit, but still enable enough air flow to pass through to create the suction effect. In general the spacings are smaller than the body size of the insects.

It is noted that a pressure gradient may be generated by one or more of multiple options. For example a blower or fan may be located below the permeable surface to suck the air. Likewise a vacuum table may be located below the surface.
The term "immobilize" as used herein means that the insects movement is substantially reduced relative to regular movement of the insect. It will be appreciated that reference is made to the average movement of the insects. There will always be insects that are able to move when other insects are completely immobilized.

The term "insects" is used generally herein. Specific reference is made to mosquitoes with the intention of covering all species of mosquitoes, in particular all species that carry disease. It is noted that one widely used application for sterilization of insects is sterilization and release of fruit flies and tsetse flies for the same purpose of insect control.

The use of a pressure gradient allows immobilization of insects to be achieved quickly, as opposed to cooling which takes time to have any effect.

In contrast with cooling, the use of a pressure gradient achieves having insects sucked towards the surface substantially immediately, that is within fractions of a seconds. Likewise, if the suction, that is the pressure gradient, is shut off, the insects resume moving immediately. Gradual changes in the suction level may be used if a more gradual effect is needed. A pressure gradient that causes an airflow through the surface of about 1m/s is sufficient to stop insects from flying. 2m/s, or 3m/s or 4m/s may stop the insects from walking, and 5m/s or 6m/s may suck them down to the surface. Exact numbers are species dependent and there is variation between individuals.

The more or less immediate response to the pressure gradient allows for improved process handling.

In one embodiment, an operator may pour mosquitoes onto the surface, and then manually manipulate the pressure gradient while examining the insects on the surface. The operator may then shut down or reduce the gradient after examination is complete to move the batch on and then repeat for a new batch. The process may be controlled by a computer or a controller using suitable software.

The present embodiments may apply an operation to an individually separated and immobile insect. In order to carry out operations in commercial quantities, initially a mass of insects is obtained. Overall, the numbers of insects may number in the hundreds, thousands or even millions, although dealt with in batches, but even with smaller batches the numbers are far too large for individual attention by humans. Thus the mass of insects is directed to one side of a permeable surface and a pressure gradient is applied across the permeable surface. The surface may be taut netting held on a frame or may be a porous surface, and the pressure gradient prevents the insects from flying away. The pressure gradient is lower away from the side that the sects are on, so that the insects experience being sucked down onto the surface. In one option, the pressure gradient may start at a relatively low level so that the insects may be unable to fly but are able to walk, thus untangling themselves from the mass. The gradient may then be increased to a level at which the insects are also unable to walk and thus fully immobilized. Once immobilized, one or more operations are carried out on the immobilized insects, as will be discussed below.

The pressure gradient may come from suction from the lower side of the permeable surface or may come from overpressure from the upper side where the insects are located.

The permeable surface may be all or part of a conveyor moving the insects between two different locations, and one or more operations may be carried out at various points along the travel of the conveyor. The pressure gradient may be applied along the length of the travel of the conveyor and may be applied by a series of fans spread out along the length which may be set to a single setting or may be set independently to allow varying pressure gradient along the conveyor.

Once the insects are separate from each other and immobile it is possible to use imaging to obtaining an image and determine a position of an individual insect. It is also possible to count the insects. If it is desired to sterilize the insects then a preset radiation dosage may be applied to an individual insect, the coordinates of the insect being known. The radiation dosage may be obtained by focusing a beam from a radiation source onto the individual insect or towards the insect presumed location or area. The strength of the beam and the time may be controlled to give the required dosage as accurately as possible. The dosage may be applied all at once or several applications of radiation may be made at separate intervals.

The operation may include determining a sex of an individual insect, and this may be done by recognition algorithms based on an obtained image. The operation may remove an individual insect if determined to be of a first sex and/or irradiating the individual insect if determined to be of a second sex. Thus males may be irradiated and females may be disposed of.

Instead of applying a focused beam onto an individual insect, it may be possible to apply an unfocused radiation dose substantially evenly over the permeable surface to reach each of the separated insects.

The mass of insects may be obtained say by feeding newly emerged adults into an airflow, say through a duct to fly to a collection area.

Alternatively, the mass of insects may be collected from conveyors or directly from containers by pouring when immobilized, either by cooling or by using an anaesthetizing agent. In such a case the insects may be warmed to remobilize the insects and allow separation, prior to gradually increasing the pressure to immobilize them again once separated. Warming may involve active warming, or passive warming, meaning simply waiting after the mosquitoes are transferred from the cold area to the warm area.

The operation, whether counting, classification, sorting or sterilization, may be applied at an operation station. The insects may be conveyed up to the operation station and then held stationary on a stationary surface for application of the operation. For example the insects may be conveyed on a plate made of the permeable surface and then when reaching the operation station the plate may be shunted aside from the conveyor to carry out the operation while stationary. Alternatively, the operation may be applied as the insects, immobile on the plate, are conveyed with the moving plate past the operation station.

The operation may involve sterilization, and a sterilization beam may be swept over the plate across said insects. The sweeping may be carried out by electronically steering the radiation beam or it may be carried out by moving the radiation source. For example a radiation source may be mounted for x and y movement over the operation station. Alternatively, multiple radiation sources may be mounted on a frame.

The operation may include classification. As discussed, an algorithm may classify an insect according to sex. A confidence score may be applied to the classification and if the score is below a predetermined threshold then the particular insect may be referred for verification to a human operator.

A graphical user interface may be used to display respective classifications to a human operator for verification, and the human operator may use the graphical user interface to apply decisions and verifications. Thus, as discussed above, the operation may involve removing an individual insect if determined to be of a first sex and/or irradiating the individual insect if determined to be of a second sex. A decision as to remove or irradiate a given insect may be applied through an interaction of the operator via the graphical user interface.

In greater detail, the best stage in which to irradiate the mosquito so as to carry out sterilization but with minimal effects on its competitiveness is during adulthood, after emergence from the pupa stage. The earlier in its life cycle that the radiation is applied, the more somatic damage is done.

In the following, embodiments are described for irradiating adult mosquitoes utilizing radiation (X Rays or Gamma rays) on a surface with immobile (stationary) mosquitoes standing on it. The immobilization (stationary phase) of the mosquitoes is achieved using a pressure difference between the two sides of the surface on which the insects are presented. As secondary means for supporting the stationary phase (or characteristic) of the mosquitoes, low temperature may be applied to the surface, either by applying a controlled low temperature. A temperature of 6-8 Celsius is recommended, but a range of 4 - 12 Celsius may still be effective. A temperature lower than 4 Celsius may harm the mosquito, the exact number depending on the species, and above 12 degrees there is almost no effect on rendering the mosquito stationary. Temperature may be controlled by blowing cooled air, or by lowering the temperature of the surface on which the mosquitoes are standing.

The application of a pressure difference to the surface between the lower and upper sides of the surface may render insects located on the surface incapable of flying away from the surface. That is to say the pressure difference and the suction they experience or endure, prevents them from flying away from the surface.

The power level of the pressure difference may be regulated to achieve an optimum between the mosquitoes not being to fly away on the one hand and on the other hand to achieve to varying extents the ability to control whether the mosquitoes can move at all, depending on the specific operation on the mosquitoes at the time. Such control can be connected to a controller connected with a camera which can identify the phase of the mosquitoes. Another level of pressure may provide that the mosquitoes are stationary, not moving, but are still standing on their legs, while for different applications, the pressure difference is sufficiently high that they are unable to stand. Standing insects are easier to classify for sex. Thus one embodiment may set a pressure that allows them to stand, for classifying, and then the pressure is increased to ensure that the insects do not move prior to performing the next operation. The level of immobility may be detected as the mosquitoes are or are not moving, for example using a computer that may analyze images using classical image difference techniques to detect difference in the image correlated with movement. Upon detection, the system may provide a trigger or send commands to increase the pressure difference if decreased immobility is required. Alternatively, upon detection the system may send requests for manual intervention. Such a process and method may be useful for example during the process when mosquitoes are being brought into contact with said surface. For example, if cold mosquitoes are poured or otherwise fall from a storage compartment, are spilled out of a storage compartment, etc. onto the surface, then while waiting for the mosquitoes to recover then either a decreasing pressure difference may be applied, or in another embodiment, a gradually increasing pressure difference may be applied. Continued cooling may be applied or not as an additional way of controlling the movement of the insects.

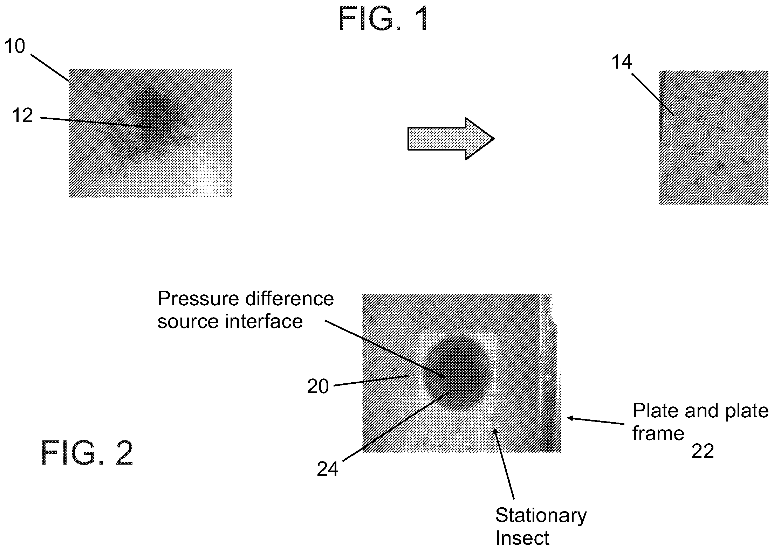

Reference is now made to Fig. 1, which illustrates a process starting point and end point. A group 10 of cold mosquitoes is located on a surface 12, and may have been poured for example. Once the temperature increases, the awakened mosquitoes may begin to travel on the surface, and they slowly detach from one another and move apart since awake mosquitoes do not naturally tend to like being on top of each other provided there is sufficient space. A camera, preferably viewing from above, records the mosquitoes on the surface either as a video or as a sequence of still images. Increasing differences between consecutive images is used to indicate that the insects are waking up and starting to move. Accordingly, a trigger operates the pressure difference mechanism to create suction that begins gently enough to enable the mosquitoes to travel and detach, and increases until reaching a maximum allowed value which may have been preset. The gradual raising of the pressure allows the mosquitoes an opportunity to spread over the surface, as shown at 14 after pouring and before immobilization takes effect. At that point, the pressure difference is such that while being fully awake (not knocked down), they cannot fly away because of the pressure difference.

An alternative version of the process may dispense with a camera, but may gradually increase the pressure difference according to a pre-calibration formula, e.g. linear with the time, until reaching a set target pressure difference.

Pressure difference for all embodiments may be generated by either only operating suction from the opposite side of the surface on which mosquitoes are located, or a combination of suction on one side and blowing air towards the surface from the other side or only blowing air towards the surface.

The suction source may be located on the opposite side of the surface to the location of the mosquitoes. The surface material may be characterized by an ability to enable air flow through the material while still able hold the insects to the material and keep them alive. Such material may be a dense net with mesh spacing of 0.12mm for example made of carbon fiber.

Referring now to Fig. 2, a porous material such as net 20 is placed within a frame 22, over duct 24 below the surface connected to a suction unit not shown. The duct may be a regular vent, axial extractor fan, a ducted fan etc.

As discussed, a group of insects, awaken, but while they are awake they are stationary because of the pressure difference between the side where they stand and the surface below the net.

Embodiments may thus provide a method for immobilizing adult mosquitoes, by providing adult mosquitoes that are located on a surface; applying a pressure difference from a suction source across the surface, so that the mosquitoes experience suction towards the opposite side of the surface to prevent them from escaping from the surface boundaries.

The surface on which the mosquitoes are located may be porous, enabling flow of air towards the suction unit on the opposite side. The suction source may be any kind of suction device such as extractor fan, axial fan. The source may be an overpressure device on the same side as the mosquitoes, such as a blower blowing air. Alternatively, a combination of both suction from the opposite side of the mosquitoes and blowing of air on the surface where the mosquitoes are standing may be used.

The adult mosquitoes may be awake mosquitoes or knocked-down mosquitoes or a combination.

Applying the pressure difference may re-regulated by a controller as discussed above to gradually increase the pressure difference by increasing the suction power on the opposite site of the surface to a maximum pre-defined value.

An apparatus for immobilizing the awake adult mosquitoes and carrying out the method just described may be provided.

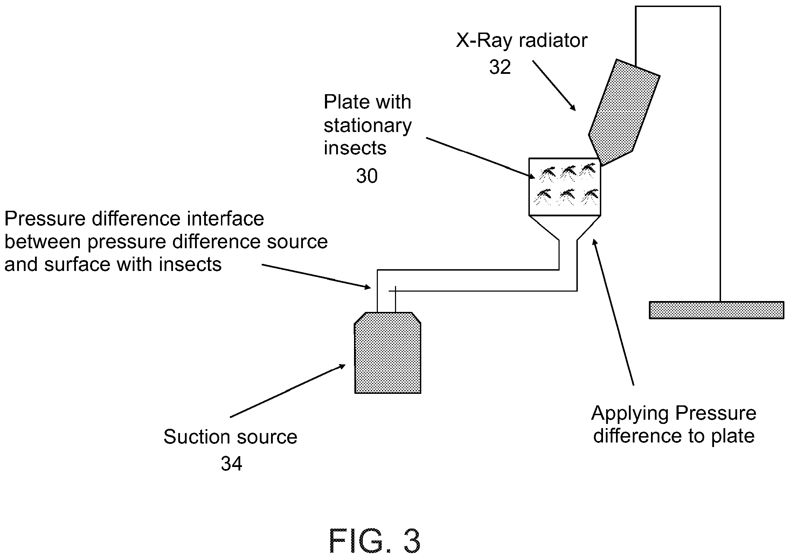

Reference is now made to Fig. 3, which illustrates how radiation may be applied to the immobilized insects. Once the mosquitoes are stationary on surface 30, irradiation may be applied, from radiation source 32, while the insects are immobilized under the influence of suction source 34. An X-Ray radiator is given merely as an example, but other kinds and sources of radiation may be applied as described below including gamma rays.

Fig. 3 thus shows apparatus for irradiating immobile but awake adult mosquitoes on a surface.

The mosquitoes may be brought to the irradiation location using one of a number of methods. These methods include spilling or pouring cold mosquitoes from a conveyor or from a container, and/or by directing mosquitoes by air flow, and even by attracting the mosquitoes using some kind of bait).

Once at the irradiation location the mosquitoes are rendered stationary, for example using the method discussed above, of applying a pressure difference on the netting or plate or other flat surface on which the mosquitoes are standing. As discussed, suction from the lower side or overpressure from the upper side or a combination of both may be used.

The idea is that the mosquitoes are manipulated to stand still on the surface, such that their vertical distance from an X-Ray irradiator is similar so that all receive a broadly similar dose and can all be given a dose which renders them sterile but does not do any additional damage and allows them to compete with wild males. Such a radiation dosage may differ between mosquito species, and an example is 60-75gy for *Aedes Albopictus.*

In yet another embodiment, instead of applying high doses directly towards the insects, fractional radiation doses but in multiple sessions may be applied to the insects in order to absorb the required amount. Fractional amounts may be supplied by the same radiation source, and the surface on which the insects are located may move, and additional fractional dosages may be applied by additional radiation sources located along the path.

An X Ray machine emitting 12gy may deliver 70gy to the surface in some 6-7 fractional doses.

The above fractional dosage method has an advantage in that less shielding is needed, since the dose at any session is lower.

Without making sure the mosquitoes are stationary, in the same posture and preferably position, the fractional radiation approach is less preferred and may yield greater variation, because if the mosquitoes are able to move in between irradiations, then different mosquitoes may eventually receive different dosages.

Another advantage of the fractional method is that the irradiation source may be particularly close to the insects on the surface on which they are located. This is specially for the case in which a robotic arm is used, in which the irradiation source is mounted on a movable controlled unit which can move along and around the surface (or the area, or the volume) being irradiated.

In another embodiment, the radiating source is a linear accelerator X Ray machine radiating onto the surface. Insects are not moving on the surface and there are no elements between the radiator unit and the insects. In one case, multiple Linear Accelerator units may be used together to cause X Ray radiated beams to converge so as to increase the dose delivery and reduce the required time for the dose delivery.

In another embodiment, once the mosquitoes are stationary, sex classification of the mosquitoes on the surface is performed in order to send the coordinates of the class of mosquitoes that is required to be removed from the surface, preferably using suction elements. The remaining insects may then be irradiated.

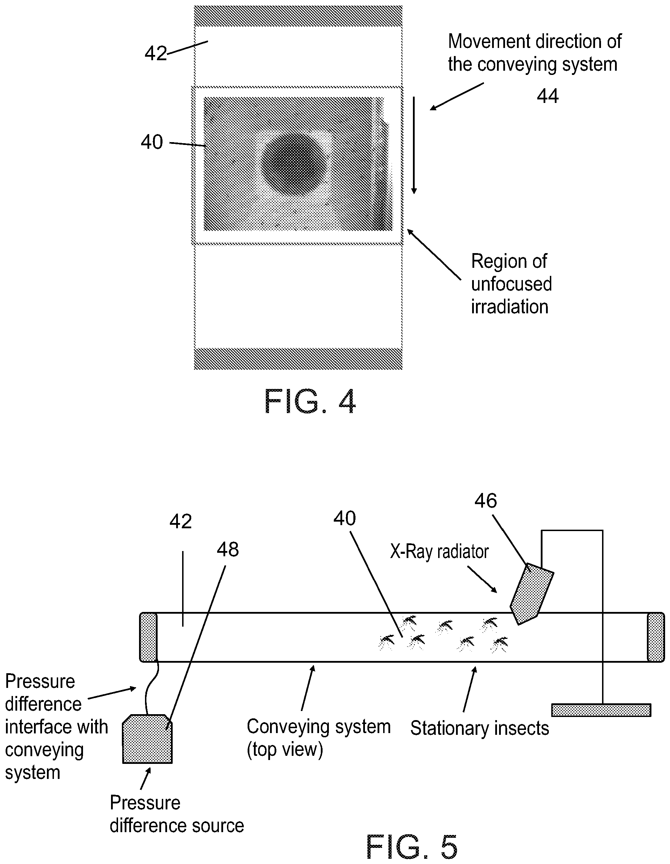

In an embodiment, enhanced uniform distribution of the radiation on the mosquitoes over the surface may be achieved by a combination of both focusing the radiation beams on the surface, and then moving the radiating elements or moving the surface, so that focused beams are able to scan the surface with the same dose being delivered to all insects, as illustrated schematically in Fig. 4 in which insects on netting 40 held on conveyor 42 are irradiated by moving the conveyor 42 in the direction of the arrow 44 and not focusing the beam so that radiation is distributed over the surface.

Specifically, the plate 40 holding the mosquitoes may be part of a conveying system including conveyor 42, and the irradiator is located at a specific point above the plate, namely the sterilization station. In such an embodiment the pressure difference may be applied along the entire conveying system.

The plate is moved under an X-Ray irradiator, or alternatively the X-Ray irradiator is moving around and along the plate, irradiating the mosquitoes standing on the plate.

As illustrated, the mosquitoes are standing and are separated from each other rather than being piled on top of one another. A constant distance between the mosquitoes on the plate and an irradiator source from above or towards one side may be maintained. If the mosquitoes are on top of one another, then the upper mosquitoes may shield the lower mosquitoes, thus increasing dosage variation.

Fig. 5 illustrates a side view of the embodiment of Fig. 4, and shows how to sterilize continuous incoming insects utilizing X-Ray. Suction source 48 is located below the insect-carrying surface, and irradiation source 46 is above the surface.

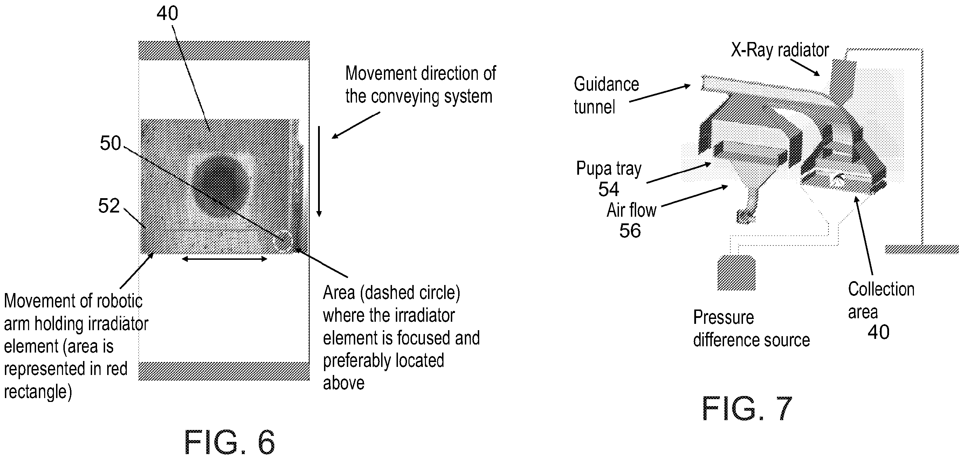

In another embodiment, the irradiating source is an X Ray Linear Accelerator machine. Fig. 6, depicts how a focused beam of radiation is applied to a single area. The focused beam is indicated in hashed lines at 50 and the sweep of the beam by the solid outline at 52, showing how the beam may sweep out an area. Combined with moving the surface by a conveying system, all insects on the conveying surface experience similar dose levels.

Referring now to Fig. 7, the mosquitoes arriving at the conveyor may be mosquitoes that have hatched as adults from their pupa at a pupa tray 54, and may have being guided using air flow 56, whether suction or blowing, from their hatching location towards a collection area, such as the flat porous surface 40. The plate may be conveyed towards the sterilization area, or the irradiation may occur in the area of the surface 40 where the mosquitoes have flown to. A direct path between the beam and the surface may be achieved by having an opening (not depicted in the drawing) that may be constantly open or may be shuttered to be open only when needed.

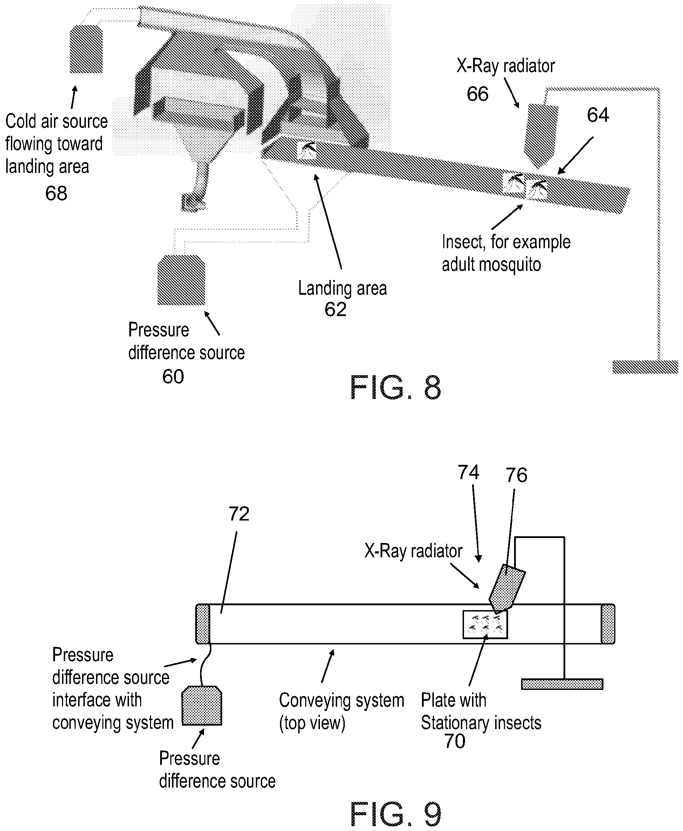

Referring now to Fig. 8, the hatching mosquitoes are guided by air, due to a pressure difference caused by suction source 60, and fly while awake towards landing area 62, and then being conveyed towards the radiation station 64 in proximity to radiation source 66. Cold air source 68 may cool the landing area as a support for the pressure difference source 60.

The system may provide a continuous flow of stationary insects towards the irradiator. The mosquitoes land while awake on the surface and therefore distance themselves from each other rather than piling up, ensuring a fixed distance between the radiator source and the insects being irradiated.

Reference is now made to Fig. 9, in which the insects land on a movable plate 70, and the movable plate may be conveyed by conveyor 72 towards irradiating station 74. The movable plate provides the ability to have a queue of surfaces with stationary insects waiting to be irradiated, and may provide an option to irradiate stationary mosquitoes which are at the same distance from the radiation source 76 because the surface or the radiation source may move so that the vertical distance to individual insects is similar. There may be a shielding (enclosure) around the entire system, or around the conveying system and the irradiating source, which is not depicted in the drawing. Such shielding may also be the shielding of the room in which the device is located.

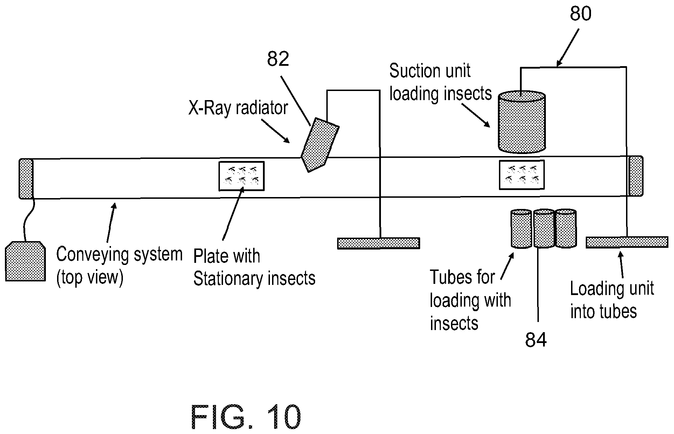

Referring now to Fig. 10, and an automatic loading robot 80 may be located after the irradiation station 82, utilizing suction or other blowing means in order to guide the irradiated insects towards storage boxes or tubes 84. An exemplary storage tube has an opening on one side to allow entry of the insects, and a closure on the other side to ensure the insects remain within. Such a closure may include a net to enable air flow out of the tube while the insects remain. As a further option, storage tubes may be located on the conveyor with the applied pressure difference, so and mosquitoes are blown into each tube. After a closure is placed on the tubes, manually or robotically, then the pressure may be turned off, allowing the insect to fly within the tube.

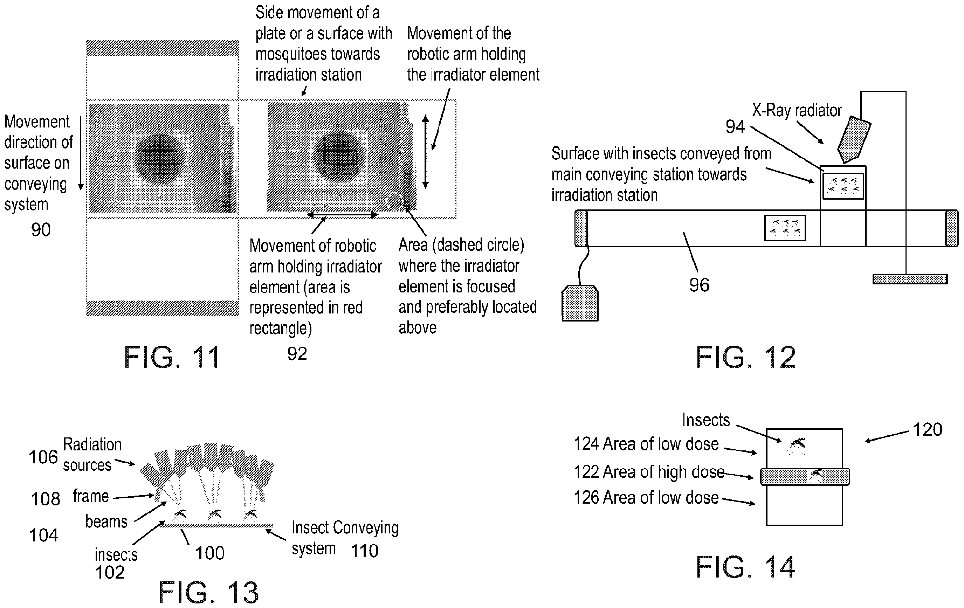

Referring now to Figs. 11 and 12, the surface on which the insects are located can be guided towards irradiation stations in the direction of arrow 90 but may also move in a direction perpendicular to the conveying direction - to give sweep 92, by using an off-the-shelf conveyor system for changing conveying direction. The advantage is that in this embodiment, the conveying system can continue conveying, while the surface being irradiated can be independently stopped and irradiated.

In an embodiment, the surface on which the insects are located may be transported to one side to be irradiated at an off conveyor location 94 - as shown in Fig. 12, and then transported back onto the main conveying system 96.

As discussed, the plate may be a sparse or dense net mounted on a frame, or it may alternatively be constructed from multiple small plates connected together and with spaces between to allow for air flow. Alternatively, the plates may be made of porous material to enable air flow, or at least to support the creation of the required pressure difference between the two sides.

If the X-Ray irradiator is not mobile, then the plate on which the mosquitoes are located may be of such a size that the radiation is almost uniformly distributed. For example, a beam of 10cm diameter would use a 10cm plate.

Moving the X-Ray radiator mechanically along the X-Y plane above the plate is possible, resulting in a more homogenous distribution of the radiation.
Alternatively or in addition, radiation may be applied above the pupa tray where mosquitoes are hatching, specifically above pupa that are emerging, or towards adults that have just emerged and are standing still on the water.

Radiation may alternatively be applied at the pupa stage, having the movable X-Ray irradiator held at a fixed distance above the pupa inside a pupa tray and irradiating the pupa on the surface.

The radiation source and beam may then be focused on a small area in the order of 1mm, so that each area may receive the same dose.

Radiation may, as mentioned, be applied by other means such as Gamma rays.

In another embodiment sex separation may be involved either prior to or following the sterilization process; Mosquitos are held stationary on the surface due to pressure difference, and a camera may take pictures of the mosquitoes. A computer algorithm may identify the sex of the individual mosquitoes and a robotic arm removes or kills the females. The robotic arm may use suction to pick up the females, and it will be appreciated that the suction is strong enough to work against the pressure gradient being used to immobilize the mosquitoes.

It is possible that for mosquitoes for which the algorithm is unable to determine the sex definitively, that is above a certain confidence level, in one embodiment such insects are irradiated and in another embodiment they are removed, the latter helping to reduce the time utilized by the irradiating system.

In an embodiment, the X-Ray or gamma ray irradiator may be robotic, being able to move above the plates or conveying system, in order to expedite the process.

A benefit of using Linear Accelerator (LINAC) technology is that LINAC's is a well proved technology for the delivery of high-energy x-rays or electrons to precise locations, as is used in radiotherapy for treating tumors.

Today there are various methods and commercial products in order to optimize the way a dose is being delivered, including positioning applicators behind the patient organ being irradiated in order to create a focused field of radiation. Particular commercial products include the CyberKnife robotic system, Gamma Knife system and other products for radiation therapy.

Radiosurgery or stereotactic radiosurgery, and radiotherapy differ by the intensity and duration of the radiation treatments, and such manipulations are suggested in the present embodiments.

In one embodiment, the method for focusing radiation beams is utilized to bring a precise dose to the surface where the adult stationary mosquitoes are located, and as the surface moves, a continuous flow of insects are moving with that surface and are irradiated with a similar dose. In an embodiment, the X ray machine includes a linear accelerator which enables focusing of the X Ray radiation towards a specific confined area of the surface where the stationary mosquitoes are located.

In an embodiment the radiating source is a source with focusing capability for focusing the radiating beams to centimeter or sub centimeter resolution, for example a linear accelerator, or gamma knife technology.

Referring now to Fig. 13, a surface 100 has stationary insects 102. The insects 102 may stationary due to a low temperature applied to their environment, and or due to pressure difference applied to the surface on which they are located. Multiple beams - hashed lines 104 - from sources 106, converge on areas on the surface which may be small enough to define individual insects, resulting in larger dose delivery to that area, and a much reduced time for irradiating of individual adult insects. The radiation sources 106 may be mounted on a frame 108. Such an embodiment also has the advantage of making it easier to provide equal dose level delivery to different insects. The surface may by moveable on a conveying system 110 or may be stationary.

Fig. 14 is a top view of a conveying system 120 with a rectangular area 122 that receives a relatively high radiation dose to sterilize insects by focusing and/or converging multiple beams. The areas 124, 126, to the sides of rectangle 122 are of lower dose. That is to say the area outside the beam focus area gets a lower dose than with unfocused systems. This ensures that insects outside the high dose area do not get too much radiation, and yet the high dose beams are still able to achieve rapid sterilization.

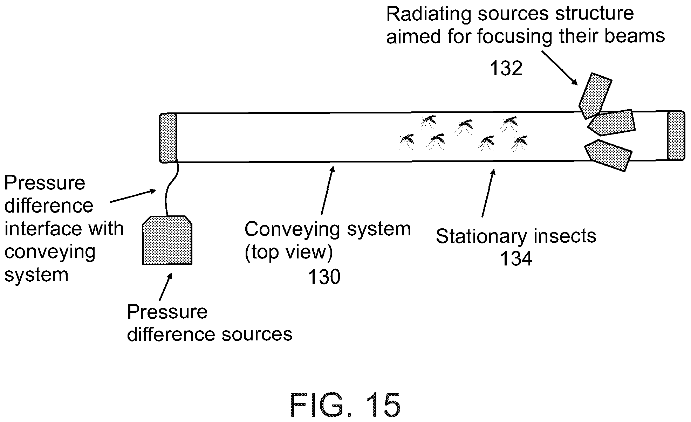

Reference is now made to Fig. 15, which is an example of a conveying system 130 with radiation sources 132 mounted so as to create a beam convergence area.

A number of different beams converge or are focused or collimated on multiple convergence areas on a surface where stationary insects 134 wait for sterilization. The surface on which the insects are stationary moves below the converging beams, hence at any moment, while each beam is of low dose, the convergence creates a high dose area for sterilization of the insects heading thereto.

Having the beams converge may provide another advantage in that insects away from the convergence area receive only a lower dose, hence both the competitiveness and longevity are less affected than when applying beams which are not focused beams on an area.

In another embodiment that uses focusing beam technology the achieved accuracy of the beam and dose delivery to the surface where the insects are located can be on the sub centimeter to sub millimeter level. Hence, moving the irradiator via a robotic arm from side to side of the conveying system perpendicular to the propagation direction of the conveying system may ensure that each insect, with length being of the order of 2-5mm, may be treated with a similar dosage.

Focusing a multiple number of beams from radiating sources such as X-Ray or Gamma ray produced by Cobalt-60, into a focused beam to deliver a higher dose to the coordinates where insects are stationary located, may expedite the delivery time of the right dose to the insects - or the area on which the insects are located - being sterilized.

In another embodiment, mosquitoes may arrive at a conveying surface from a hatching area, and above the conveying surface a sprayer may spray the conveying surface or the insects with larvicide material that mosquitoes can further deliver to other mosquitoes, specifically female mosquitoes when mating, so such wild females may transfer the larvicide to the water in which they lay their eggs, thus killing larva mosquitoes in the water.

In one embodiment, a camera is located above the conveying surface. The camera identifies coordinates of the insects on the moving conveying system. The coordinates are transferred to a radiation source, and the radiation source irradiates towards the coordinates as indicated by the camera. Radiation may be X-Ray or Gamma ray, and the source of the X-Rays may be a linear accelerator in order to provide better accuracy in delivery of dose to specific coordinates.

As mentioned, the radiation may be applied by multiple beams converging at precise locations to thereby increase the dose. Gamma rays may be focused using Gamma Knife radiosurgery technology. The mosquitoes on the radiated surface, may be adults after emerging from pupae and may be guided towards the moving surface. The mosquitoes may be males, and may be of the species *aedes albopictus, aedes aegypti, anopheles gambiae,* or any other mosquito species.

In an alternative of the above embodiment, the same may be carried out on pupae. In such a case the pupae are transported via the conveying system. The pupae may be placed in a material soaked material or they may be conveyed in a tray with shallow water that keeps them wet and immobile.

Moving on to classifying and sorting, and a person has the advantage of being able to classify visual content after very short training yet with extremely high accuracy. There are subtle visual differences between male and female mosquitoes, but while it would require lots of training and algorithm work to have a computer automatically identify the mosquito sex with high accuracy (higher than 99%), for a person these differences can be taught in a relative short time, while still resulting in high accuracy.

On the other hand, a computer program coupled with an imaging system has the advantage over a person that it is able to automatically detect many objects in a single frame very quickly, and able to determine and provide their coordinates.

An embodiment may provide a system and a method that combines those advantages into a single solution, having the advantage of a person able to classify a single mosquito with very high accuracy after a short learning period, and the computer having the ability to detect multiple objects within an image and provide their coordinates or cropped images very quickly (less than a second).

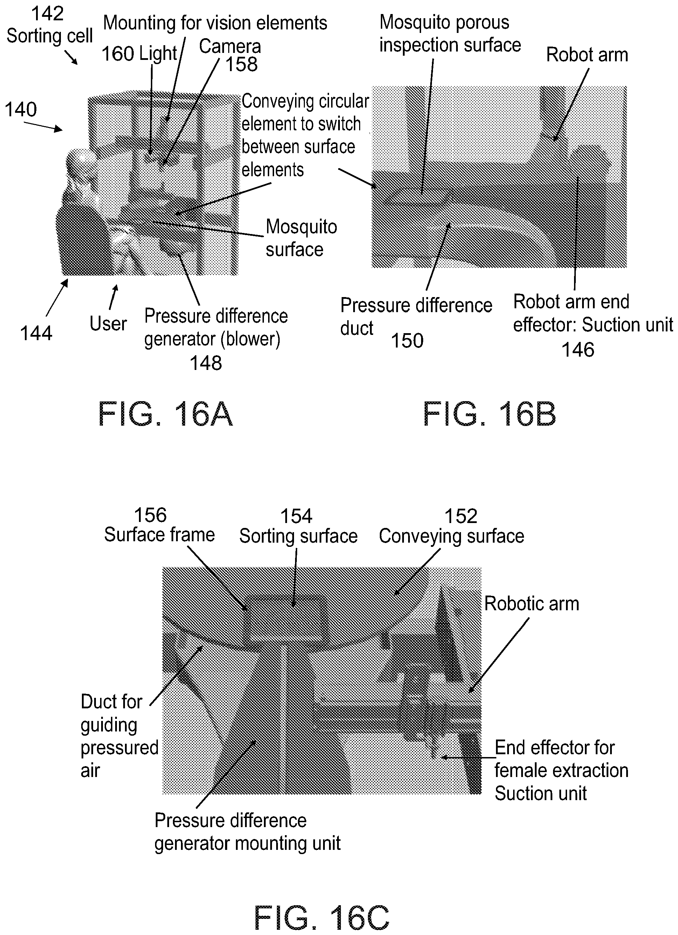

Reference is now made to Figs. 16A, 16B and 16C, which show such a combined system 140.

The combined system 140 includes a counting, sorting and classification cell 142, an operator station 144, an optional robotic end effector 146 to extract females, such as a suction pipette in this example, a pressure difference generator 148, a duct 150 for air to create pressure difference, a conveying surface 152 to convey mosquitos or to convey surfaces on which mosquitoes are located towards a classification station, a sorting surface 154 bounded by a surface frame 156, an imaging system including one or more cameras 158 and a light component 160, a light controller to control light intensity and means to control camera frames per second imaging rate, a screen with graphical user interface enabling the interaction of the user with the robotic operation of the end effector and the imaging system and a controller (e.g. a computer) that enables control of the end effector movement and extraction operation and an imaging operation to image the mosquitoes.

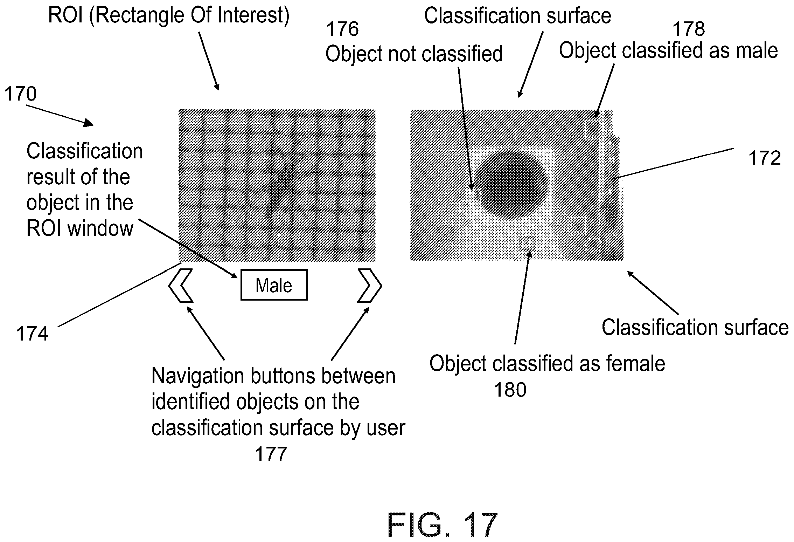

Reference is now made to Fig. 17, which illustrates a graphical user interface (GUI) 170 for interaction between the user and the system. The interface includes a screen which provide information for the user to interact with software, and the user interaction is then translated into movements of the robotic end effector for the extraction of the females:
The user interacts with the system and the robotic arm using the GUI which may include a first window 172 showing a surface with the insects located thereon, such as mosquitoes - males and females. A second window (ROI window) 174 shows an enlargement or zoom in of objects the user is exploring in the first window.

The user may manipulate a cursor in the first window, and in the second window the software crops and enlarges a rectangle with sufficient resolution and zoom in to image a single mosquito that the cursor has identified in the first window and which the software then recognizes. The rectangle size would typically represent a 5mm over 5mm square for the aedes albopictus mosquitoes, while it can also be 10mm or over 10mm for example. The rectangle may be set for different sizes, depending on the typical size of the object (or mosquito) of interest being explored (or classified).

The following is a description of how the solution may work and enable a collaborative process for sex sorting.

Initially, an operator places, or supervises automated placing of, a new batch of immobile mosquitoes on a surface located externally to the robotic operational area. In the case of non-automated placing, the external area may ensure safety of the person operating the system.

The surface loaded with mosquitoes is conveyed towards the classification station under the camera. Conveying operates such that the surface on which the mosquitoes are located moves into the machine and towards the classification station, located adjacent and preferably under the imaging system, thus the camera and light elements.

At the classification station, a pressure difference is applied as discussed above.
The pressure difference may be set to ensure there is no damage to the mosquitoes. The loaded surface is now inside the classification station, and object detection may begin once the mosquitoes are immobile.

The software may automatically start as it detects insertion of a new surface with a new batch of insects. Thus, classical object detection algorithms may identify a sequence of having no mosquitoes correlating to moving out the previous surface, and then having a new set of mosquito objects under the camera, and such a sequence may trigger a detection algorithm. Alternatively, the operator may provide an indication. The indication may be part of or may trigger another screen in which various parameters may be set.

The software may automatically detect any mosquito objects on the porous surface. Thus object detection algorithms may detect mosquitoes located on the surface. The information pertaining to the objects is provided to the user and displayed according to rules.

Mosquito objects that are not classified yet by their gender are annotated by shape and color of type A, for example dashed square 176 in Fig. 17.

In a semi-automatic mode, the user may travel between the identified objects by clicking on navigation buttons 177. The next insect appears in the ROI window.

In parallel, annotation is provided on the Classification surface screen (the first window) to show the user which object it is now looking at. The shape for example may remain the same but the color may change, or the insect may flash or any other change may be used that will easily identify to the operator which object is now being focused on.

For each mosquito, the user may choose, using a classification interaction, if the object is a female or male. A button is used by the user to provide the software with the result decided by the user. The button may be annotated by the text "Female", but any other text or shape, color can be applied for the design of such button as long as it differentiates and makes it clear that the reference is to a female or a male. In one example the button may use the international icon for female and female, or may have two different shapes and the user may click on the appropriate icon, or there may be a single button which indicates being selected so that the result is the text of the selected button. If the button is not selected then the result is the opposite, e.g. male.

Alternatively the button may contain an indicator of the relevant action. For example, the text can sate the word "KILL", or "EXTRACT". The button may thus constitute an operation interaction.

The operator may explore each object, or the algorithm may suggest in a variance of the solution to send pictures of the objects only when the algorithm accuracy for classifying the object is below a specific threshold.

The computer and the object detection and or classification algorithms may be remotely located away from the pressure difference surface and communication may be sent over the internet or other means of communication.

Mosquito objects that are classified as males may be marked on the first window showing all objects with shape and color of type B, 178, and the other mosquitoes will be annotated as type C 180.

The interaction over the GUI is then translated to movements of the robot in the sorting cell.

The robotic arm may move and extract each female, and hence perform a sex sorting process, after each decision that identifies the object in the ROI as a female. Alternatively, a multiple set of decisions involving a queue of female coordinates on the porous surface may be acted on together, and the robotic arm will move between the coordinates to extract the females one after the other while operating the suction unit only when the end effector is located above the suspected female.

The robotic arm may be a suction pipette or other end effector that can functionally remove the females. Alternatively, a laser may transmit a beam to each coordinate, based for example on a galvanometer.

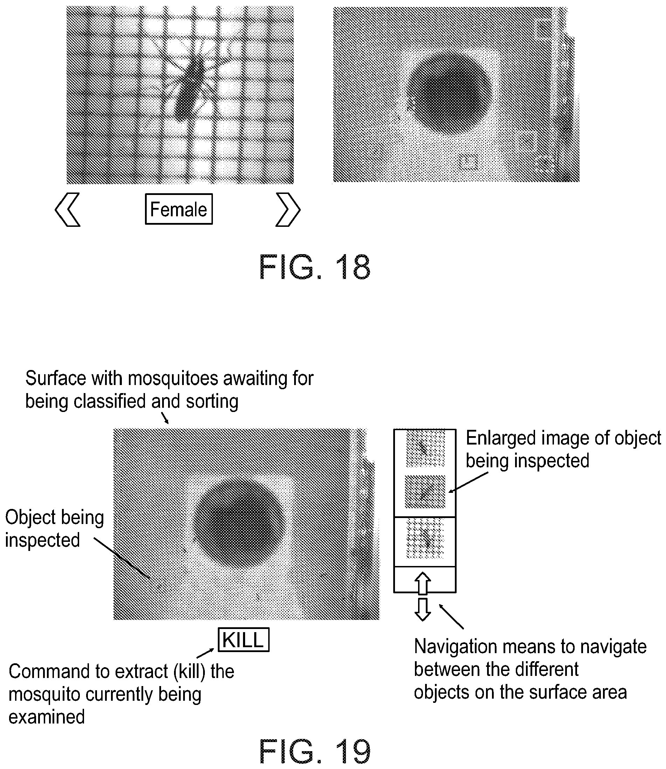

Referring now to Fig. 18, the operator has explored another mosquito from the first window which is now zoomed in on the second window.

Reference is now made to Fig. 19, which shows a GUI using different text and labels, while keeping the same functionality. The GUI includes two sections, a first window in which the entire surface on which the mosquitoes are located is present, and a second window, which may include more than one image. The second window provides the ability to inspect a single mosquito, and to navigate between the different objects in the first window.

Instead of clicking on "female", in this example the user simply clicks on a KILL button, meaning the mosquito now being inspected is a female and upon pressing "KILL" either the robotic end effector immediately extracts it from the surface, or the software controller may add it to a queue of jobs for female extraction, so that after finishing the sequential classification of the all the objects it may sequentially extract all the objects identified as females.

In a variation of the above, when applying the mosquitoes onto the surface, it is possible to pour out a large group of immobile mosquitoes, for example cold mosquitoes. In such a case, the mosquitoes may happen to be one on top of the other, and or tangled to each other in such that when being imaged by the imaging system, it is not possible to differentiate between the different mosquito objects.

The present embodiment may untangle the mosquitoes by placing the immobile group of mosquitoes on a surface that is able to support a pressure difference, such as the porous surfaces described above. The group of mosquitoes may be set to be active again, for example by increasing the temperature to above 12 degrees Celsius (preferably above 20 degrees Celsius being relative active) inside the sorting cell, or by simply waiting until room temperature is reached and not activating a cooling system. Increase in temperature may alternatively involve increasing the temperature of the surface itself on which the mosquitoes are located.

Once the mosquitoes became active, they untangle from each other and move apart from each other. Then, once the mosquitoes are identifiable as single objects, a second immobilization is applied by introducing the pressure difference to the surface on which the insects are located or lowering the temperature inside the sorting cell so that the mosquitoes may substantially stop moving, as discussed above typically between 6-8 degree Celsius for *aedes albopictus.*

Once the mosquitoes are immobile, the semi-automatic process is applied.

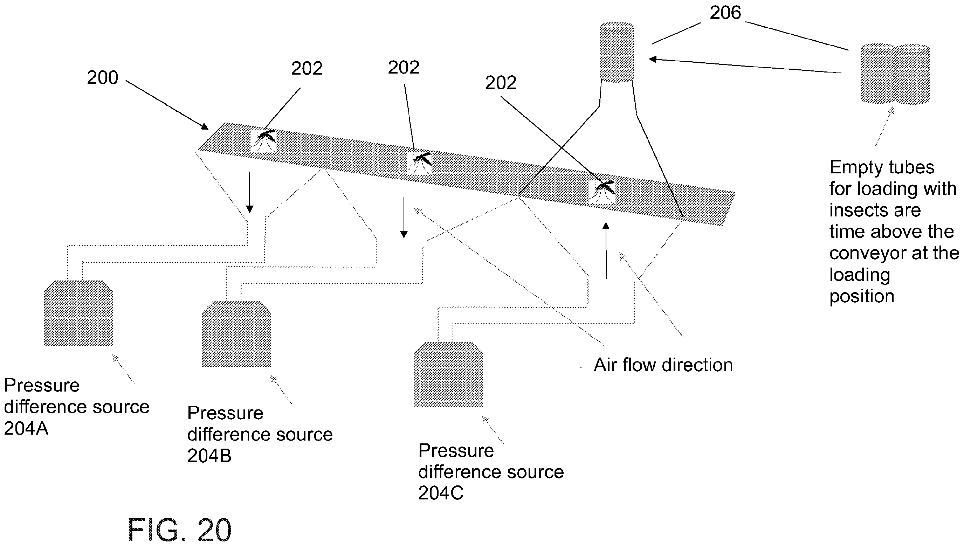

Reference is now made to Fig. 20, which illustrates an embodiment of the present invention in which the permeable surface is part of a moving conveyor 200, thus forming a porous conveyed surface, or permeable conveyed surface. The conveyor carries the mosquitoes from one end to the other. The permeable surface may be the entire conveyor or may comprise discrete inserts 202 in the conveyor which carry the insects. The insects are conveyed along the conveyor and the pressure gradient is applied along the length along which the insects are conveyed. In many cases a single pressure source may not be sufficient to maintain pressure along the full length of the conveyor and, in the case illustrated, three pressure sources 204A, 204B and 204C are used. The different pressure sources may be at different settings. For example a high setting may be used on the landing area, a lower setting in a travel area and a third setting which is operation dependent may be used in an operating area.

At the end of conveyor 200, the insects are loaded into storage tubes 206.

The terms `classification surface' and `sorting surface' may refer to the same physical surface in the drawings above.

The present embodiments may thus provide a system that includes software, an imaging system, a classification surface for holding the mosquitoes on the surface, and a pressure difference generator intended for making the mosquitoes substantially immobile by having them not able to move because of the suction forces on the porous surface.

The Semi-automatic sorting method and system include placing adult mosquitoes on a surface, such as a porous surface. A pressure difference is applied to the porous surface, causing the mosquitoes on the surface be substantially immobilized.

Imaging may then be carried out of the immobile mosquitoes on the surface, and object detection may identify locations of the mosquitoes. The mosquitoes on the surface may be projected onto a screen. An operator may annotate the objects as male or female. The result together with coordinates may be sent to the system controller, and a robotic element may extract females based on user feedback and coordinates.

After finishing sorting, the plate with mosquitoes may be taken away for packing the sorted mosquitoes and the process begins again with a new set of unsorted mosquitoes.

It is also possible that the person may also perform the object detection and explore for any female mosquitoes on the computer screen, and an advantage of having the classification by the person is to eliminate any potential machine classification errors.

The surface on which the mosquitoes are standing may be fixed, or may be movable. If the surface is fixed, mosquitoes are placed each time on the fixed surface.

Placing the mosquitoes on the surface, either as the surface is moving or if it is fixed in position may involve pouring mosquitoes onto the surface, or having adults fly and be guided towards the surface.

For the classification of the mosquito gender, the classification is based on visual differences between the male and females features, and the imaging system may include a light and a lighting control system. In an embodiment the entire classification and sorting is automatic, and the computer classifies the mosquito sex on its own using image processing algorithms. In such case, the pressure difference is used to immobilize the mosquitoes, and once they are positioned under the camera, they are classified and later sorted automatically.

It is expected that during the life of a patent maturing from this application many relevant imaging, object recognition, beam focusing and irradiation systems will be developed and the scopes of the corresponding terms are intended to include all such new technologies *a priori.*

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Citation or identification of any reference in this application shall not be construed as an admission that such reference is available as prior art to the present invention. To the extent that section headings are used, they should not be construed as necessarily limiting.

## Claims

1. A method of immobilizing and applying an operation to insects, comprising:
obtaining insects;
directing said insects to a first side of a permeable surface (12);
applying a pressure gradient across said permeable surface (12), to provide a pressure which is lower at a second side of said surface than at said first side, said pressure gradient thereby being effected as a pull of said insects to said permeable surface to immobilize said insects on said permeable surface;
conveying said insects on said permeable surface under continuing application of said pressure gradient;
and
carrying out said operation on said immobilized insects.

2. The method of claim 1, wherein said applying a pressure gradient comprises applying suction across said permeable surface from said second side, or wherein said applying a pressure gradient comprises adjusting between a first pressure gradient and a second pressure gradient, said second pressure gradient being steeper than said first pressure gradient, said first pressure gradient allowing insects to walk and separate from each other but not fly, and said second pressure gradient preventing said insects from walking.

3. The method of claim 1 or claim 2, wherein said permeable surface is at least part of a moving conveyor (42) having a conveying length along which said insects are conveyed, the pressure gradient being applied along said conveying length, and/or applying pressure gradients at respective locations along said conveying length, said pressure gradients at said respective locations differing from one another, and/or applying a third pressure gradient, said third pressure gradient being higher than said first pressure gradient and lower than said second pressure gradient, and/or wherein any one of said pressure gradients is reversible in direction.

4. The method of any one of the preceding claims, wherein said operation comprises counting insects on said permeable surface and/or applying a larvicide, and/or obtaining one member of the group consisting of an image and a position of an individual insect, and/or applying a predetermined radiation dosage to an individual insect and/or focusing a beam from a radiation source (34) onto said individual insect according to an obtained position, and/or determining a sex of an individual insect, and/or removing an individual insect if determined to be of a first sex and/or irradiating said individual insect if determined to be of a second sex, and/or applying an unfocused radiation dose evenly over said permeable surface to reach each of said separated insects.

5. The method of any one of the preceding claims, wherein said obtaining said insects comprises causing said insects to fly through a duct (24) with an airflow to a collection area and/or said obtaining said insects comprises collecting said mass of insects when immobilized by cooling or using an anaesthetizing agent, and/or wherein when said mass of insects are immobilized by cooling, warming said insects to remobilize said insects and allow separation, prior to said applying said pressure gradient.

6. The method of any one of the preceding claims, comprising applying said operation at an operation station, said insects being conveyed up to said operation station and then kept stationary for said operation or comprising applying said operation at an operation station while said insects are conveyed past said operation station.

7. The method of any one of the preceding claims, wherein said operation comprises sterilization, the method comprising sweeping a sterilization beam across said insects and/or wherein the operation comprises classification, the method comprising using an algorithm to classify an insect according to sex, applying a score to said classification and if said score is below a predetermined threshold then referring said insect to a human operator, and/or wherein the operation comprises classification, the method comprising using an algorithm to classify an insect according to sex, and using a graphical user interface to display respective classifications to a human operator for verification, and/or wherein said operation comprises removing an individual insect if determined to be of a first sex and/or irradiating said individual insect if determined to be of a second sex and a decision regarding said removing or irradiating is obtained via said graphical user interface, and/or wherein the operation comprises sterilization, said sterilization comprising moving at least one radiation source (34) in two dimensions in a plane above said permeable surface, and/or wherein the operation comprises sterilization, said sterilization comprising moving permeable surface, and/or herein the operation comprises sterilization and said predetermined dose is provided in a plurality of fractional doses with intervals therebetween.

8. The method of claim 1,
wherein said pressure gradient comprises:
applying a first pressure gradient across said collecting surface to allow individual insects to separate from said mass but not to fly away; and
applying a second pressure gradient, said second pressure gradient being higher than said first pressure gradient, to said insects to immobilize said insects.

9. Apparatus for carrying out an operation on insects, comprising:
a permeable surface (12) for receiving said insects;
a pressure source (204) for applying a pressure gradient across said permeable surface;
a pressure source controller for controlling said pressure gradient between a first level at which said insects on said permeable surface are able to walk but unable to fly and a second level being higher than said first level, in which said insects are immobilized; and
an operating station for carrying out an operation on said insects while immobilized.

10. Apparatus according to claim 9, further comprising a camera (158) for obtaining images and/or coordinates of said insects and/or a robot actuated device for removing identified ones of said insects based on provided coordinates, and/or at least one radiation source (34) configured to provide a predetermined radiation dose to a specified location, and/or comprising a graphical user interface (170), the graphical user interface providing interactions to allow an operator to control said operations, and/or comprising a graphical user interface (170), the graphical user interface providing interactions to allow an operator to control said operations, and wherein said graphical user interface comprises:
a first view showing an area of said permeable surface with insects labeled according to a classification or as unclassified and allowing for selection between said insects; and
a second view showing an enlargement of a selected insect in said first view; and/or
at least one classification or counting interaction to input a classification of a currently selected insect; and/or
said graphical user interface comprises
at least one operation interaction to apply an operation to a currently selected insect.

11. Apparatus according to claim 9 wherein:
said permeable surface (12) is conveyable along a length of travel, for receiving said insects;
said pressure source(204A) is at a first location on said permeable surface for applying a pressure gradient across said permeable surface; and
a second pressure source (204B) is located at a second location on said permeable membrane or at least one duct (24) is provided for conducting air pressure to different locations along said length of travel.

12. The method of claim 1 wherein:
said insects are obtained via a duct (24);
said directing said insects to a first side of a permeable surface comprises using a first pressure source; and
said applying a pressure gradient through said permeable surface comprises using a second pressure source.

13. The apparatus of claim 9, wherein:
the permeable surface (12) is located on a conveyor (42);
the pressure source (204) being configured to apply said pressure gradient through said permeable surface to be lower at a second side of said permeable surface than at said first side, thereby to immobilize said insects on said permeable surface, wherein the immobilized insects are conveyed on said permeable surface to said operating station.

14. The apparatus of claim 9, wherein:
the first pressure source (204) is configured to apply a first pressure gradient across said permeable collecting surface to immobilize said insects, the pressure gradient being applied such that the collected insects are pressured into the surface; and
the permeable collecting surface (12) is part of a conveyor (42) for conveying said insects on said permeable surface to a location for carrying out said operation on said insects.

15. The apparatus of claim 14, comprising:
a duct (24) for supplying of said insects leading to said permeable surface; and
a second pressure source, (204B) associated with said duct to guide insects through said duct to a first side of said permeable surface, said first side facing said duct.

## Patentansprüche

1. Verfahren zur Immobilisierung und Anwendung einer Operation an Insekten, umfassend:
die Beschaffung von Insekten;
Lenken der Insekten auf eine erste Seite einer durchlässigen Oberfläche (12);
Anlegen eines Druckgradienten über die durchlässige Oberfläche (12), um einen Druck bereitzustellen, der an einer zweiten Seite der Oberfläche niedriger ist als an der ersten Seite, wobei der Druckgradient dadurch als ein Sog der Insekten zu der durchlässigen Oberfläche bewirkt wird, um die Insekten auf der durchlässigen Oberfläche zu immobilisieren;
Fördern der Insekten auf der durchlässigen Oberfläche unter fortgesetzter Anwendung der Druckgradienten;
und
Durchführen die Operation an den immobilisierten Insekten.

2. Verfahren nach Anspruch 1, wobei das Anlegen eines Druckgradienten die Anwendung einer Saugwirkung über die durchlässige Oberfläche von der zweiten Seite umfasst, oder wobei das Anlegen eines Druckgradienten das Einstellen zwischen einem ersten Druckgradienten und einem zweiten Druckgradienten umfasst, wobei der zweite Druckgradient steiler als der erste Druckgradient ist, der erste Druckgradient, wobei der erste Druckgradient es den Insekten ermöglicht, zu laufen und sich voneinander zu trennen, aber nicht zu fliegen, und der zweite Druckgradient die Insekten am Laufen hindert.

3. Verfahren nach Anspruch 1 oder Anspruch 2, wobei die durchlässige Oberfläche zumindest Teil eines sich bewegenden Förderers (42) ist, der eine Förderlänge aufweist, entlang der die Insekten gefördert werden, wobei der Druckgradient entlang der Förderlänge angelegt wird, und/oder Druckgradienten an jeweiligen Stellen entlang der Förderlänge angelegt werden, wobei sich die Druckgradienten an den jeweiligen Stellen voneinander unterscheiden, und/oder Anlegen eines dritten Druckgradienten, wobei der dritte Druckgradient höher als der erste Druckgradient und niedriger als der zweite Druckgradient ist, und/oder irgendeiner der Druckgradienten in der Richtung umkehrbar ist

4. Verfahren nach einem der vorhergehenden Ansprüche, wobei die Operation das Zählen von Insekten auf der durchlässigen Oberfläche und/oder das Aufbringen eines Larvizids und/oder das Erhalten eines Mitglieds der Gruppe, die aus einem Bild und einer Position eines einzelnen Insekts besteht, und/oder das Anwenden einer vorbestimmten Strahlungsdosis auf ein einzelnes Insekt und/oder das Fokussieren eines Strahls von einer Strahlungsquelle (34) auf das einzelne Insekt gemäß einer erhaltenen Position umfasst, und/oder das Bestimmen eines Geschlechts eines einzelnen Insekts und/oder das Entfernen eines einzelnen Insekts, wenn festgestellt wird, dass es ein erstes Geschlecht hat, und/oder das Bestrahlen des einzelnen Insekts, wenn festgestellt wird, dass es ein zweites Geschlecht hat, und/oder das gleichmäßiges Anwenden einer nicht fokussierten Strahlungsdosis über die durchlässige Oberfläche, um jedes der getrennten Insekten zu erreichen.

5. Verfahren nach einem der vorhergehenden Ansprüche, wobei die Beschaffung der Insekten das Veranlassen der Insekten, durch einen Kanal (24) mit einem Luftstrom zu einem Sammelbereich zu fliegen, umfasst und/oder die Beschaffung der Insekten das Sammeln der Insektenmasse umfasst, wenn diese durch Kühlen oder unter Verwendung eines Betäubungsmittels immobilisiert ist, und/oder wobei, wenn die Insektenmasse durch Kühlen immobilisiert ist, die Insekten erwärmt werden, um die Insekten zu remobilisieren und eine Trennung zu ermöglichen, bevor der Druckgradient angelegt wird.

6. Verfahren nach einem der vorhergehenden Ansprüche, bei dem die Operation an einer Arbeitsstation durchgeführt wird, wobei die Insekten bis zu dieser Arbeitsstation befördert werden und dann für die Operation stationär gehalten werden, oder bei dem die Operation an einer Arbeitsstation durchgeführt wird, während die Insekten an der Arbeitsstation vorbeigeführt werden.

7. Verfahren nach einem der vorhergehenden Ansprüche, wobei die Operation die Sterilisation umfasst, wobei das Verfahren das Überstreichen der Insekten mit einem Sterilisationsstrahl umfasst, und/oder wobei die Operation die Klassifizierung umfasst, wobei das Verfahren die Verwendung eines Algorithmus zur Klassifizierung eines Insekts gemäß dem Geschlecht umfasst, das Anwenden einer Punktzahl auf die Klassifizierung und, wenn die Punktzahl unter einem vorbestimmten Schwellenwert liegt, das Weiterleiten des Insekts an einen menschlichen Bediener, und/oder wobei die Operation eine Klassifizierung umfasst, wobei das Verfahren das Verwenden eines Algorithmus zum Klassifizieren eines Insekts nach dem Geschlecht und das Verwenden einer grafischen Benutzerschnittstelle zum Anzeigen der jeweiligen Klassifizierungen an einen menschlichen Bediener zur Verifizierung umfasst, und/oder wobei die Operation das Entfernen eines einzelnen Insekts umfasst, wenn festgestellt wird, dass es einem ersten Geschlecht angehört, und/oder das Bestrahlen des einzelnen Insekts, wenn festgestellt wird, dass es einem zweiten Geschlecht angehört, und eine Entscheidung bezüglich des Entfernens oder Bestrahlens über die grafische Benutzerschnittstelle erhalten wird, und/oder wobei die Operation eine Sterilisation umfasst, wobei die Sterilisation das Bewegen mindestens einer Strahlungsquelle (34) in zwei Dimensionen in einer Ebene über der durchlässigen Oberfläche umfasst, und/oder wobei die Operation eine Sterilisation umfasst, wobei die Sterilisation das Bewegen von mindestens zwei Strahlungsquellen (34) umfasst, um einen fokussierten Strahl oder einen homogenen Strahl auf der durchlässigen Oberfläche bereitzustellen, und/oder wobei die Operation eine Sterilisation umfasst und die vorbestimmte Dosis in einer Vielzahl von fraktionierten Dosen mit Intervallen dazwischen bereitgestellt wird.

8. Verfahren nach Anspruch 1, wobei der Druckgradient Folgendes umfasst:
Anlegen eines ersten Druckgradienten über die Sammelfläche, um es einzelnen Insekten zu ermöglichen, sich von der Masse zu trennen, aber nicht weg zu fliegen; und
Anlegen eines zweiten Druckgradienten, wobei der zweite Druckgradient höher ist als der erste Druckgradient, an die Insekten, um die Insekten zu immobilisieren.

9. Vorrichtung zum Anwendung einer Operation an Insekten, umfassend:
eine durchlässige Oberfläche (12) zum Aufnehmen der Insekten;
eine Druckquelle (204) zum Anlegen eines Druckgradienten über die durchlässige Oberfläche; eine Druckquellensteuerung zum Steuern des Druckgradienten zwischen einem ersten Niveau, bei dem die Insekten auf der durchlässigen Oberfläche laufen aber nicht fliegen können, und einem zweiten Niveau, das höher ist als das erste Niveau, bei dem die Insekten immobilisiert werden; und
eine Arbeitsstation zum Anwenden einer Operation an den Insekten, während sie immobilisiert sind.

10. Vorrichtung nach Anspruch 9, die ferner eine Kamera (158) zur Aufnahme von Bilder und/oder Koordinaten der Insekten und/oder eine robotergesteuerte Vorrichtung zum Entfernen der identifizierten Insekten auf der Grundlage der angegebenen Koordinaten, und/oder mindestens eine Strahlungsquelle (34), die so konfiguriert ist, dass sie eine vorher festgelegte Strahlungsdosis an eine spezifizierte Stelle bereitstellt, und/oder eine grafische Benutzerschnittstelle (170) umfasst, wobei die grafische Benutzerschnittstelle Interaktionen bereitstellt, um es einem Bediener zu ermöglichen, die Vorgänge zu steuern, und/oder eine grafische Benutzerschnittstelle (170) umfasst, wobei die grafische Benutzerschnittstelle Interaktionen bereitstellt, um es einem Bediener zu ermöglichen, die Vorgänge zu steuern, und wobei die grafische Benutzerschnittstelle umfasst:
eine erste Ansicht, die einen Bereich der durchlässigen Oberfläche mit Insekten zeigt, die gemäß einer Klassifizierung oder als unklassifiziert gekennzeichnet sind, und die eine Auswahl zwischen den Insekten ermöglicht; und
eine zweite Ansicht, die eine Vergrößerung eines ausgewählten Insekts in der ersten Ansicht zeigt; und/oder die grafische Benutzerschnittstelle ferner umfasst:
mindestens eine Klassifizierungs- oder Zählinteraktion, um eine Klassifizierung eines aktuell ausgewählten Insekts einzugeben; und/oder die grafische Benutzerschnittstelle umfasst:
mindestens eine Operationsinteraktion, um eine Operation auf ein aktuell ausgewähltes Insekt anzuwenden.

11. Vorrichtung nach Anspruch 9, bei der:
die durchlässige Oberfläche (12) entlang einer Bewegungslänge förderbar ist, um die Insekten aufzunehmen;
die Druckquelle (204A) sich an einer ersten Stelle auf der durchlässigen Oberfläche befindet, um einen Druckgradienten über die durchlässige Oberfläche anzulegen; und
eine zweite Druckquelle (204B) sich an einer zweiten Stelle auf der durchlässigen Membran befindet oder mindestens ein Kanal (24) vorgesehen ist, um den Luftdruck zu verschiedenen Stellen entlang der genannten Bewegungslänge zu leiten.

12. Verfahren nach Anspruch 1, wobei:
die Insekten über einen Kanal (24) erhalten werden;
das Lenken der Insekten auf eine erste Seite einer durchlässigen Oberfläche die Verwendung einer ersten Druckquelle umfasst; und
das Anlegen eines Druckgradienten durch die durchlässige Oberfläche die Verwendung einer zweiten Druckquelle umfasst.

13. Vorrichtung nach Anspruch 9, wobei:
die durchlässige Oberfläche (12) auf einem Förderer (42) angeordnet ist;
die Druckquelle (204) so konfiguriert ist, dass sie den Druckgradienten durch die durchlässige Oberfläche so anlegt, dass er an einer zweiten Seite der durchlässigen Oberfläche niedriger ist als an der ersten Seite, um dadurch die Insekten auf der durchlässigen Oberfläche zu immobilisieren, wobei die immobilisierten Insekten auf der durchlässigen Oberfläche zu der Arbeitsstation befördert werden.

14. Vorrichtung nach Anspruch 9, wobei:
die erste Druckquelle (204) so konfiguriert ist, dass sie einen ersten Druckgradienten über die durchlässige Sammelfläche anlegt, um die Insekten zu immobilisieren, wobei der Druckgradient so angelegt wird, dass die gesammelten Insekten in die Oberfläche gedrückt werden; und
die durchlässige Sammelfläche (12) Teil eines Förderers (42) ist, um die Insekten auf der durchlässigen Oberfläche zu einer Stelle zu befördern, an dem die Operation an den Insekten durchgeführt wird.

15. Vorrichtung nach Anspruch 14, umfassend:
einen Kanal (24) zur Versorgung der Insekten, der zu der durchlässigen Oberfläche führt; und
eine zweite Druckquelle (204B), die mit dem Kanal verbunden ist, um Insekten durch den Kanal zu einer ersten Seite der durchlässigen Oberfläche zu leiten, wobei die erste Seite dem Kanal zugewandt ist.

## Revendications

1. Méthode d'immobilisation et d'application d'une opération sur des insectes, comprenant :
obtenir des insectes ;
diriger lesdits insectes vers un premier côté d'une surface perméable (12) ;
appliquer un gradient de pression à travers ladite surface perméable (12), afin de fournir une pression qui est plus faible sur un deuxième côté de ladite surface que sur ledit premier côté, ledit gradient de pression étant ainsi effectué comme une traction desdits insectes vers ladite surface perméable afin d'immobiliser lesdits insectes sur ladite surface perméable ;
transporter lesdits insectes sur ladite surface perméable sous l'application continue dudit gradient de pression ;
et
réaliser l'opération sur les insectes immobilisés.

2. Méthode de la revendication 1, dans laquelle l'application d'un gradient de pression comprend l'application d'une aspiration à travers la surface perméable depuis ledit deuxième côté, ou dans laquelle ladite application d'un gradient de pression comprend l'ajustement entre un premier gradient de pression et un deuxième gradient de pression, ledit deuxième gradient de pression étant plus pentu que ledit premier gradient de pression, ledit premier gradient de pression permettant aux insectes de marcher et de se séparer les uns des autres mais pas de voler, et ledit deuxième gradient de pression empêchant lesdits insectes de marcher.

3. Méthode de la revendication 1 ou de la revendication 2, dans laquelle ladite surface perméable est au moins une partie d'un convoyeur mobile (42) ayant une longueur de transport le long de laquelle lesdits insectes sont transportés, le gradient de pression étant appliqué le long de ladite longueur de transport, et/ou en appliquant des gradients de pression à des emplacements respectifs le long de ladite longueur de transport, lesdits gradients de pression auxdits emplacements respectifs différant l'un de l'autre, et/ou en appliquant un troisième gradient de pression, ledit troisième gradient de pression étant plus élevé que ledit premier gradient de pression et plus bas que ledit deuxième gradient de pression, et/ou dans lequel l'un quelconque desdits gradients de pression est réversible dans le sens.

4. Méthode selon l'une quelconque des revendications précédentes, dans laquelle ladite opération consiste à compter les insectes sur ladite surface perméable et/ou à appliquer un larvicide, et/ou à obtenir un membre du groupe consistant en une image et une position d'un insecte individuel, et/ou à appliquer un dosage de rayonnement prédéterminé à un insecte individuel et/ou à focaliser un faisceau d'une source de rayonnement (34) sur ledit insecte individuel en fonction d'une position obtenue, et/ou déterminer un sexe d'un insecte individuel, et/ou retirer un insecte individuel s'il est déterminé qu'il est d'un premier sexe et/ou irradier ledit insecte individuel s'il est déterminé qu'il est d'un deuxième sexe, et/ou appliquer une dose de rayonnement non focalisée uniformément sur ladite surface perméable afin d'atteindre chacun desdits insectes séparés.

5. Méthode de l'une quelconque des revendications précédentes, dans laquelle l'obtention desdits insectes consiste à faire voler lesdits insectes à travers un conduit (24) avec un flux d'air vers une zone de collecte et/ou ladite obtention desdits insectes consiste à collecter ladite masse d'insectes lorsqu'ils sont immobilisés par refroidissement ou à l'aide d'un agent anesthésiant, et/ou lorsque ladite masse d'insectes est immobilisée par refroidissement, à réchauffer lesdits insectes pour les remobiliser et permettre leur séparation, avant d'appliquer ledit gradient de pression.

6. Méthode de l'une quelconque des revendications précédentes, comprenant l'application de ladite opération à un poste d'opération, lesdits insectes étant transportés jusqu'audit poste d'opération et ensuite maintenus immobiles pour ladite opération, ou comprenant l'application de ladite opération à un poste d'opération tandis que lesdits insectes sont transportés au-delà dudit poste d'opération.

7. Méthode de l'une quelconque des revendications précédentes, dans laquelle ladite opération comprend la stérilisation, la méthode comprenant le balayage d'un faisceau de stérilisation à travers lesdits insectes et/ou dans laquelle l'opération comprend la classification, la méthode comprenant l'utilisation d'un algorithme pour classer un insecte en fonction du sexe, appliquer un score à ladite classification et si ledit score est inférieur à un seuil prédéterminé, renvoyer ledit insecte à un opérateur humain, et/ou dans laquelle l'opération comprend la classification, la méthode comprenant l'utilisation d'un algorithme pour classer un insecte en fonction du sexe, et l'utilisation d'une interface utilisateur graphique pour afficher les classifications respectives à un opérateur humain pour la vérification, et/ou dans laquelle ladite opération comprend le retrait d'un insecte individuel s'il est déterminé comme étant du premier sexe et/ou l'irradiation dudit insecte individuel s'il est déterminé comme étant d'un deuxième sexe et une décision concernant ledit retrait ou irradiation est obtenue via ladite interface utilisateur graphique, et/ou dans laquelle l'opération comprend la stérilisation, ladite stérilisation comprenant le déplacement d'au moins une source de rayonnement (34) en deux dimensions dans un plan au-dessus de ladite surface perméable, et/ou dans laquelle l'opération comprend la stérilisation, ladite stérilisation comprenant le déplacement d'au moins deux sources de rayonnement (34) pour fournir un faisceau focalisé ou un faisceau homogène sur ladite surface perméable, et/ou dans laquelle l'opération comprend la stérilisation et ladite dose prédéterminée est fournie en une pluralité de doses fractionnées avec des intervalles entre elles.

8. Méthode de la revendication 1,
dans laquelle ledit gradient de pression comprend :
l'application d'un premier gradient de pression à travers ladite surface de collecte pour permettre aux insectes individuels de se séparer de ladite masse mais pas de voler ; et
l'application d'un deuxième gradient de pression, ledit deuxième gradient de pression étant plus élevé que ledit premier gradient de pression, auxdits insectes pour les immobiliser.

9. Appareil pour effectuer une opération sur des insectes, comprenant :
une surface perméable (12) pour recevoir lesdits insectes ;
une source de pression (204) pour appliquer un gradient de pression à travers ladite surface perméable ;
un contrôleur de source de pression pour contrôler ledit gradient de pression entre un premier niveau auquel lesdits insectes sur ladite surface perméable sont capables de marcher mais incapables de voler et un deuxième niveau étant plus élevé que ledit premier niveau, dans lequel lesdits insectes sont immobilisés ; et
un poste d'opération pour effectuer une opération sur lesdits insectes pendant qu'ils sont immobilisés.

10. Appareil selon la revendication 9, comprenant en outre une caméra (158) pour obtenir des images et/ou des coordonnées desdits insectes et/ou un dispositif actionné par un robot pour retirer les insectes identifiés parmi lesdits insectes sur la base de coordonnées fournies, et/ou au moins une source de rayonnement (34) configurée pour fournir une dose de rayonnement prédéterminée à un emplacement spécifié, et/ou comprenant une interface utilisateur graphique (170), l'interface utilisateur graphique fournissant des interactions pour permettre à un opérateur de contrôler lesdites opérations, et/ou comprenant une interface utilisateur graphique (170), l'interface utilisateur graphique fournissant des interactions pour permettre à un opérateur de contrôler lesdites opérations, et dans laquelle ladite interface utilisateur graphique comprend :
une première vue montrant une zone de ladite surface perméable avec des insectes étiquetés selon une classification ou comme non classifiés et permettant une sélection entre lesdits insectes ; et
une deuxième vue montrant un agrandissement d'un insecte sélectionné dans ladite première vue ; et/ou ladite interface utilisateur graphique comprend en outre :
au moins une interaction de classification ou de comptage pour saisir une classification d'un insecte actuellement sélectionné ; et/ou
ladite interface utilisateur graphique comprend
au moins une interaction d'opération pour appliquer une opération sur un insecte actuellement sélectionné.

11. Appareil selon la revendication 9 dans lequel :
ladite surface perméable (12) est transportable le long d'une longueur de déplacement, pour recevoir lesdits insectes ;
ladite source de pression (204A) est située à un premier emplacement sur ladite surface perméable pour appliquer un gradient de pression à travers ladite surface perméable ; et
une deuxième source de pression (204B) est située à un deuxième emplacement sur ladite membrane perméable ou au moins un conduit (24) est fourni pour conduire la pression de l'air à différents emplacements le long de ladite longueur de déplacement.

12. Méthode de la revendication 1 dans laquelle :
lesdits insectes sont obtenus par un conduit (24) ;
ladite direction desdits insectes vers un premier côté d'une surface perméable comprend l'utilisation d'une première source de pression ; et
ladite application d'un gradient de pression à travers ladite surface perméable comprend l'utilisation d'une deuxième source de pression.

13. Appareil de la revendication 9, dans lequel :
la surface perméable (12) est située sur un convoyeur (42) ;
la source de pression (204) étant configurée pour appliquer ledit gradient de pression à travers ladite surface perméable pour être plus faible à un deuxième côté de ladite surface perméable qu'au premier côté, de manière à immobiliser lesdits insectes sur ladite surface perméable, les insectes immobilisés étant transportés sur ladite surface perméable jusqu'audit poste d'opération.

14. Appareil de la revendication 9, dans lequel :
la première source de pression (204) est configurée pour appliquer un premier gradient de pression à travers ladite surface de collecte perméable pour immobiliser lesdits insectes, le gradient de pression étant appliqué de manière à ce que les insectes collectés soient pressés dans la surface ; et
la surface de collecte perméable (12) fait partie d'un convoyeur (42) pour transporter lesdits insectes sur ladite surface perméable jusqu'à un emplacement où effectuer ladite opération sur lesdits insectes.

15. Appareil de la revendication 14, comprenant :
un conduit (24) pour l'alimentation desdits insectes menant à ladite surface perméable ; et
une deuxième source de pression (204B), associée audit conduit pour guider les insectes à travers ledit conduit vers un premier côté de ladite surface perméable, ledit premier côté faisant face audit conduit.
